# EUROPEAN PATENT APPLICATION

(11) **EP 2 347 706 A1**
(43) Date of publication of application: **27.07.2011**
(21) Application number: 11150784.4
(22) Date of filing: 13.01.2011
(51) Int. Cl.: A61B 5/021, G06F 19/00

(54) **Device and method for measuring the blood pressure**

(30) Priority: 14.01.2010 EP 10150739
(71) Applicant: Microlife Intellectual Property GmbH, 9443 Widnau (CH)
(72) Inventor: Frick, Gerhard, 6800 Feldkirch (AT)
(74) Representative: Hepp Wenger Ryffel AG

(57) **Abstract**

The invention relates to a device and method for determining a cardiovascular risk factor of a patient through non-invasive measurement of the blood pressure of the patient. The device (10) comprises a storage means (20) for storing results of consecutive blood pressure measurements and a processor (18) for determining if there is a trend in the blood pressure measurements. Further, a display (22) integral to the device displays the trend in the blood pressure measurements.

## Description

The invention relates to a device and method for non-invasively measuring the blood pressure of a patient.

Hypertension is increasingly viewed by the medical profession as a complex cardiovascular disorder, not simply a matter of determining whether a patient's blood pressure values are high or low relative to some accepted standard model. Specifically, a patient's blood pressure value should be viewed in the context of the individual patient's global risk, not merely compared to an expected blood pressure value range. It is increasingly recognised that identifying blood pressure trends in patients that are already diagnosed as suffering from hypertension and also patients considered to be "pre-hypertensive" (ie. individuals having one or more cardiovascular risk factors) is highly valuable for their effective treatment. A blood pressure trend refers to the overall tendency of consecutive blood pressure measurements (that are taken over a period of, for example, several days or months). Thus such a blood pressure trend may be a tendency to consistently increasing measurements or consistently decreasing measurements or measurements staying substantially constant in value.

Blood pressure measurements may also help to identify other medical conditions where hypertension arises. One example of such a condition is pre-eclampsia (also known as pregnancy-induced hypertension), which may affect pregnant women from 20 weeks after gestation. Blood pressure elevation is the most visible sign of this disease, along with significant amounts of protein in the urine.

Non-invasive blood pressure measuring devices with a wide variety of modifications and attachments are known. Such blood pressure measuring devices determine, for example, the value of the systolic and diastolic blood pressure on the basis of an oscillometric measurement. During this type of blood pressure measurement, the pressure within an inflated cuff wrapped around the arm of a patient is measured during deflation of the cuff. The systolic and the diastolic blood pressure are determined on the basis of the variation of the cuff pressure. Further references to blood pressure measurement in this description should be considered as referring to measurement of both systolic and the diastolic blood pressure.

EP 1258223 A1 discloses a method of recording and storing a series of several blood pressure measurements taken over a short term period. The stored blood pressure measurements may then be processed to provide a determination of whether the patient's blood pressure variation is normal. Such information is useful for medical professionals to analyse certain cardiovascular issues, but such short term analysis may provide an incomplete picture of the patient's cardiovascular risk.

EP 1102196 A2 discloses a system and method for producing a blood pressure measurement and then transferring the blood pressure data into a programmer. The blood pressure measurements may then be sent periodically via the internet to a server which stores the blood pressure data in a database for subsequent analysis. In these type of systems the blood pressure readings may subsequently be processed in a variety of ways to obtain average blood pressure readings or trend analyses. Generally, the server and database are located at a medical establishment, such as a doctor's surgery. Here, the processed blood pressure readings are reviewed by a medical professional and the results are discussed with the patient when they attend an appointment at the doctor's surgery.

Such systems have the disadvantage that there is inherently a time delay between the patient's blood pressure measurement being recorded and the medical professional knowing the outcome of the blood pressure measurement analysis process. The time delay may be several days or more. Further, at the time the blood pressure measurements are taken, the patient is not able to know immediately if the current trend of their blood pressure is upwards or downwards. If such information were available to the patient directly and automatically after their blood pressure reading was taken, they would have the possibility to make lifestyle adjustments in the light of that information. This also means that patient care in the home is not a possibility.

It is also time-consuming and inconvenient for a patient to download their blood pressure measurements on a daily basis to a processing device, such a personal computer.

It is an object of the present invention to overcome the drawbacks of the prior art, especially to provide a blood pressure measuring device which provides information simply and quickly to the user in order to help them review their own potential cardiovascular risk.

Another object of the invention is to provide an early information to the patient automatically, without any additional operation, if there is a risk related to his blood pressure, long before these values reaches one of the average individual defined "unhealthy" level. It is known in hypertension research, that any consistent upwards tendency over time represents a risk factor, which should be known and corrected as early as possible. These and other objects are solved according to the present invention with a method and a blood pressure measuring device according to the features of the independent claims.

Another object of the present invention is to provide an early warning to the patient if blood pressure levels show an acute hypertensive trend or if unusual tension increases are recorded over a short period of time.

According to the device of the present invention, there is provided a storage means for storing results of consecutive blood pressure measurements and a processor for determining if there is a trend in the blood pressure measurements. The storage means and processor are integral to the blood pressure measuring device, thereby avoiding separate apparatus which would be inconvenient and more expensive for the user.

The processor is designed to perform a comparison between two or more diastolic blood pressure values and similarly two or more systolic blood pressure values are compared.

The device also provides a display for displaying the trend in the blood pressure measurements. Advantageously, this display will be viewed immediately by the patient who consequently feels involved in this data collection process. The patient will immediately know if the current trend of his blood pressure is up or down and will be reminded to make suitable lifestyle adjustments. For example, if their blood pressure trend is in an upward direction, then the patient may decide to avoid a stressful activity that may increase their blood pressure reading further in the following days.

Alternatively, the device may also provide warning modes. A warning may warn the patient if the blood pressure trend shows a continuous increase over a certain period of time, when the device is in a first warning mode. A second warning may warn the patient if the blood pressure values show a high increase, e.g. of at least 30 mmHg systolic and/or at least 15 mmHg diastolic pressure, over a short period of time, for example less than 2 days, when the device is in a second warning mode. Such an increase may be an indicator for a medical condition, such a pre-eclampsia. The warning may be in the form of a special symbol on the display and/or an acoustic warning tone. The device may also provide means for switching between the first warning mode and the second warning mode.

According to the method of the present invention, in a first step a blood pressure measurement of the patient is recorded at a plurality of measurement times and in a second step consecutive blood pressure measurements are stored. This invention may be realised in a blood pressure monitor including a date and time function which enables the time and date of each blood pressure measurement to be recorded. In such a blood pressure monitor each blood pressure measurement can be stored in association with the time of recording.

Alternatively, this invention may be realised in a blood pressure monitor that does not include a date and time function and therefore it is necessary to instruct the user that one blood pressure measurement per day is to be taken at approximately the same time each day. This daily blood pressure measurement is stored in the monitor for subsequent comparison with consecutive daily blood pressure measurements.

In a third step, a trend in the blood pressure measurements is determined. If a trend can be determined, then in a fourth step the trend is displayed.

In a preferred embodiment, the daily blood pressure measurements over a predetermined period of time, such a month, are interpolated in order to obtain a trend line. If the trend line has a gradient greater than a predetermined steepness, then a trend is determined and displayed.

If several measurements are taken during the course of a day, then the average blood pressure measurement may be determined and stored in the memory as the daily blood pressure measurement.

In an alternative embodiment, the calculation of the blood pressure trend utilises the current daily blood pressure measurement and at least the one previous daily blood pressure measurement. For example, three consecutive daily blood pressure measurements can be denoted as m1, m2 and m3. Where it is determined that m1 < m2 < m3, then an upward tendency is calculated and a first symbol is displayed. Where it is determined that m1 > m2 > m3, then a downward tendency is calculated and a second symbol is displayed. Where there is no clear tendency or it is determined that the daily blood pressure measurements are substantially constant, then a third symbol, or no symbol, is displayed.

Any suitable symbols may be used to indicate the blood pressure trend. For example, an upward tendency may be indicated with an arrow pointing substantially upwards relative to the display of the blood pressure monitor. A downward tendency may be indicated by an arrow pointing substantially downwards relative to the display of the blood pressure monitor. A substantially constant result may be indicated by an arrow pointing substantially horizontally relative to the display of the blood pressure monitor or by no symbol. Alternatively, any three suitable letters, words or colours may be used.

The processor is pre-programmed with minimum variation criteria that determine when the no clear tendency result should be given. Specifically, if the difference between the systolic blood pressure measurements of m1 and m2 is +/-5mmHg or the difference between the diastolic blood pressure measurements of m1 and m2 is +/-3mmHg, then the processor determines that no clear tendency is present.

The trend will be preferably only calculated and displayed, as soon as enough data are ready in the device memory. The quality of the trend indication will increase with the number of data available. It will depend on the device memory size, and on the number of measurements taken with the device since the last memory reset. A memory reset could either be manually by a specific user operation, i.e. push of a reset button or combination of buttons, or after a battery change, in case the device is not equipped with a permanent memory.

Further, the processor may be pre-programmed to display a warning, e.g. for hypertensive development, if the calculated trend shows a continuous increase of the blood pressure values over a specific period. As an example, if the blood pressure trend shows increasing values for at least 20 consecutive days, the processor will display a warning.

As a further alternative, the processor may be pre-programmed to display a warning if blood pressure values show a high increase over a short period of time. For example, a warning may be shown if an increase of systolic pressure of more than 30 mmHg and/or of diastolic pressure of more than 15 mmHg is detected over a period of less than 2 days to warn the patient for a possible hypertensive condition, such a preeclampsiaeclampsiaeclampsiaeclampsia.

Further, a switching means may be provided to allow for switching between the two warning modes.

The invention will now be described with reference to the accompanying drawings in which:
Figure 1 is a diagram of a blood pressure measuring device of the present invention with attached cuff, and
Figure 2 is a schematic representation of the blood pressure measuring device of the present invention, and
Figure 3 is an illustration of an exemplary display of the present invention.
Figure 4 is a graph showing systolic blood pressure values (on the y-axis) for a specific patient over a time period of a month (days recorded on the x-axis).

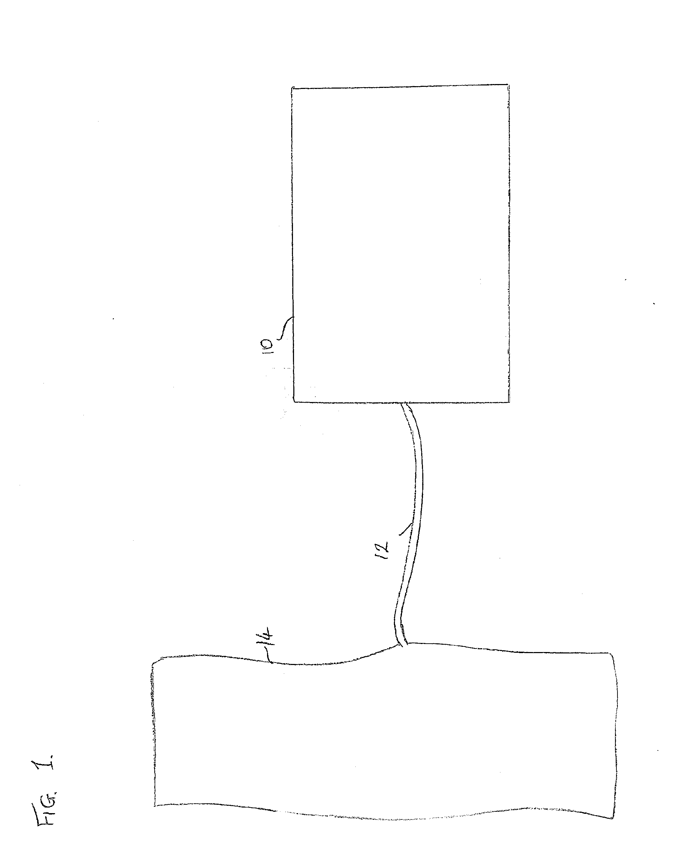

Figure 1 shows a simplified diagram of a blood pressure measuring device 10 of the present invention with an inflatable cuff 14 attached via a flexible tube 12.

In use, the cuff 14 is usually wrapped around the upper arm of a patient. The technique of inflation and gradual deflation of the cuff 14 and simultaneous measurement of pressure variations in the cuff and oscillometric determination of the blood pressure values is well-known in the art and will not be described here.

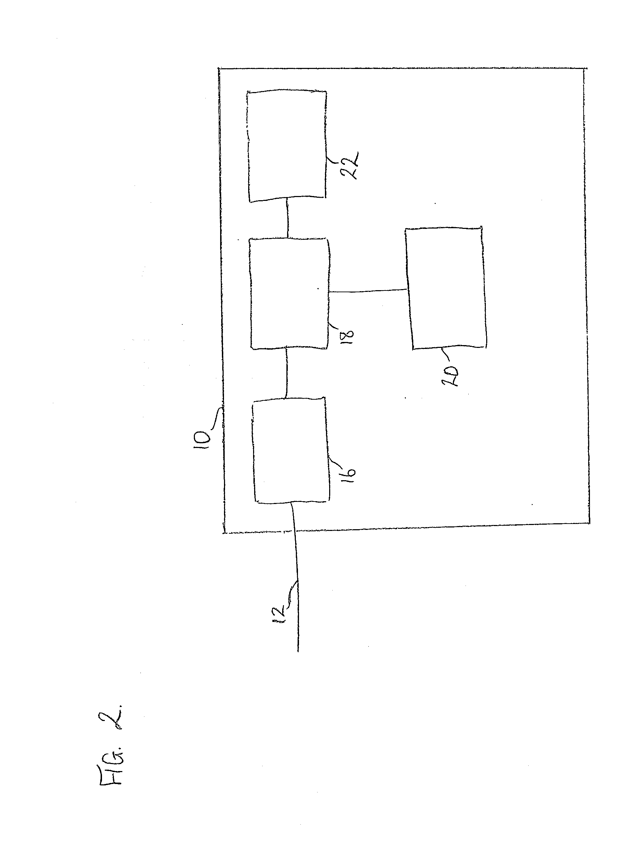

The blood pressure measuring device 10 according to the present invention is schematically shown in Figure 2. The device comprises a pressure sensor 16, a processor 18, a memory 20, a display 22 and a battery (not illustrated). The pressure sensor 16 may be an electric or electronic pressure sensor for measuring pressure within the cuff 14. Specifically, pressure oscillations in the cuff 14 measured by the pressure sensor 16 enable the systolic and diastolic blood pressures to be calculated by the processor 18 with an oscillometric algorithm. For this purpose the pressure sensor 16 is coupled to the processor 18. Further, the memory 20 and the display 22 are also connected to the processor 18. In a preferred embodiment, the processor 18 includes a date and time function.

In use, the processor 18 may be pre-programmed to execute a blood pressure measurement at a set time or times each day. The blood pressure measurement is made in a standard manner through inflation and gradual deflation of the cuff. The blood pressure calculated by the processor 18 from the information provided by the pressure sensor 16 is then stored in the memory 20 associated to date and time of measurement execution. The memory 20 may have a capacity to store up to several hundred blood pressure measurements. Where several blood pressure measurements are executed in a day, then the processor 18 determines a daily average blood pressure reading and stores this result in the memory 20 associated to the date. An algorithm to minimise the effect of artefacts in the readings is preferably utilised. For example, the daily average blood pressure reading can be determined on the basis of an algorithm described in EP 1258223 A1 (by the present applicant) which minimises the effect of artefacts and determines whether the patient is in a resting state suitable for obtaining a normal blood pressure reading.

The display 22 may be activated by a dedicated user-operated switch (not illustrated) on the blood pressure measuring device 10 or, preferably, the display 22 may automatically switch on when the cuff is inflated.

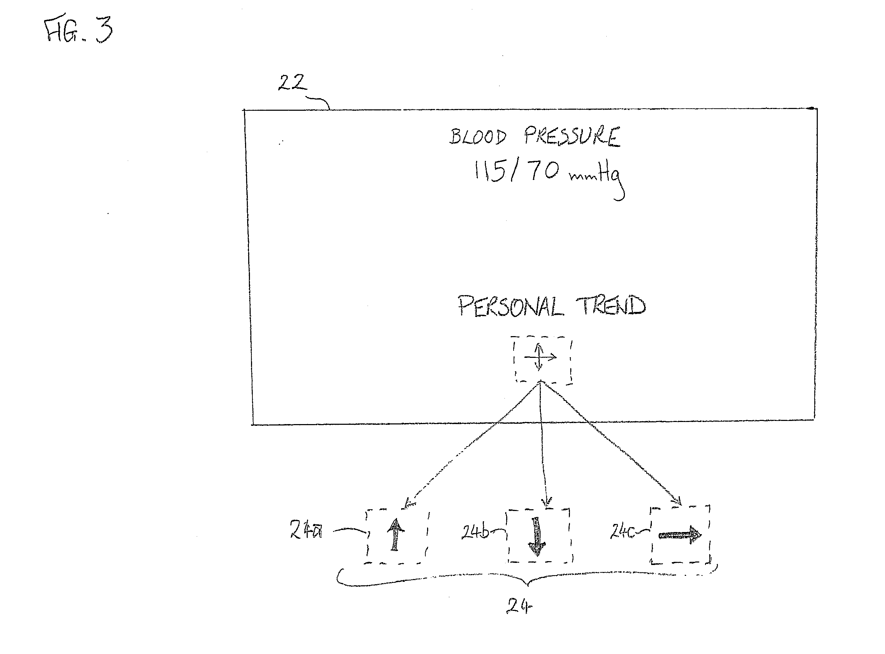

Figure 3 is an illustration of an exemplary display 22 of the present invention. The display 22 shows the current blood pressure measurement in a standard format showing the systolic and diastolic blood pressure readings. In different part of the display 22 a personal trend of the user is shown. This personal trend information is calculated in the processor 18 and in one embodiment it is determined from the current and two proceeding stored blood pressure measurements.

Regardless of the number of available data inside the memory, above a certain minimum required data amount, a linear trend line will be calculated, eg. by linear interpolation. If the trend line achieves a certain steepness over time, a trend indication will be displayed. The steepness threshold can be dynamically adjusted according to the actual number of data, for example a higher threshold will be required for a smaller number of data. The steepness threshold will be smaller, the larger the number of available values in the memory.

A minimum number of data is required in order that the personal trend calculation will be enabled. Typically data from a two week measurement period, if the devices is equipped with date and time functions. In a further embodiment the device requires a one month measurement period and thereafter calculates the trend based on the preceeding one month of daily measurements. If the device is not equipped with date and/or time function, each value will be treated as a daily value.

A users' instruction manual is provided to the user for explanation of the specific use of the device.

Preferably, the personal trend will be calculated for systolic and diastolic pressure separately. If one or both values fulfil the threshold steepness criteria, a personal trend will be indicated. The gradient of the trend line can be different for the systolic pressure and diastolic pressure separately. For example, if more than one personal trend calculations are controversial, no personal trend will be indicated.

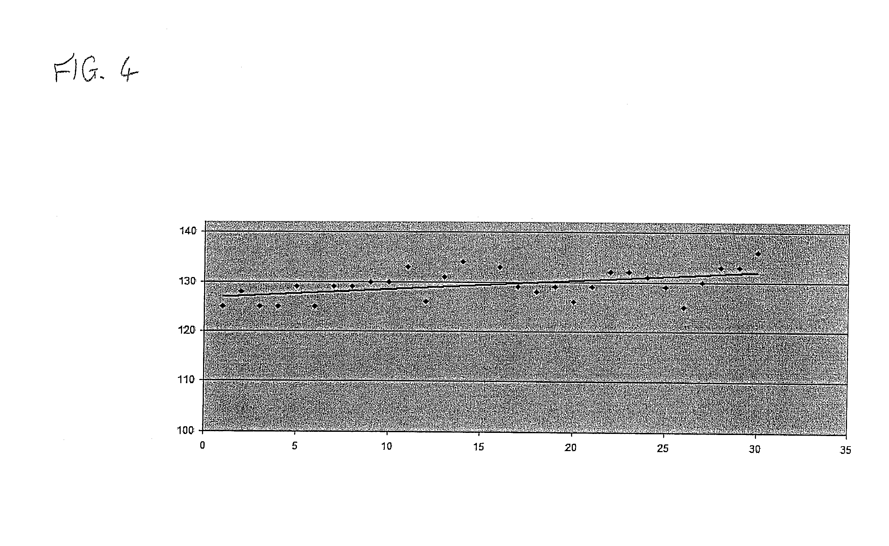

Figure 4 is a graph showing systolic blood pressure values (on the y-axis) for a specific patient over a time period of a month (days recorded on the x-axis). The solid diagonal line shows the personal trend of the patient for the last 30 days, which is an increasing systolic blood pressure trend.

In a further embodiment, the device does not require any setting and does not provide different modes for the personal trend calculation. The personal trend calculation will be adjusted automatically, depending on the blood pressure data available in the memory. In an exemplary embodiment, a minimum number of blood pressure readings in order to determine a personal trend is 14 readings. In this embodiment, if a patient's systolic blood pressure values have a variation of more than 5mmHg over a period of 30 days, then the personal trend of the patient would be calculated and displayed on the device. If a patient's systolic blood pressure values have a variation of less than 5mmHg over a period of 30 days, then the personal trend of the patient would be not be calculated and the device would indicate that no trend is present.

In alternative embodiments having a higher minimum number of blood pressure readings, then the minimum variation in a patient's blood pressure readings over a predetermined period of days can be lower and still the trend would calculated and indicated on the device.

In a preferred embodiment the personal trend information 24 is displayed using arrow symbols 24 to indicate an upwards, downwards or consistent blood pressure trend. With reference to Figure 3, an upward tendency is indicated with an arrow 24a pointing substantially upwards relative to the display of the blood pressure monitor. A downward tendency is indicated by an arrow 24b pointing substantially downwards relative to the display of the blood pressure monitor. A substantially constant result is indicated by an arrow 24c pointing substantially horizontally relative to the display of the blood pressure monitor 10. When no symbol is shown on the display following a blood pressure measurement, this indicates that inadequate data is stored in the device memory.

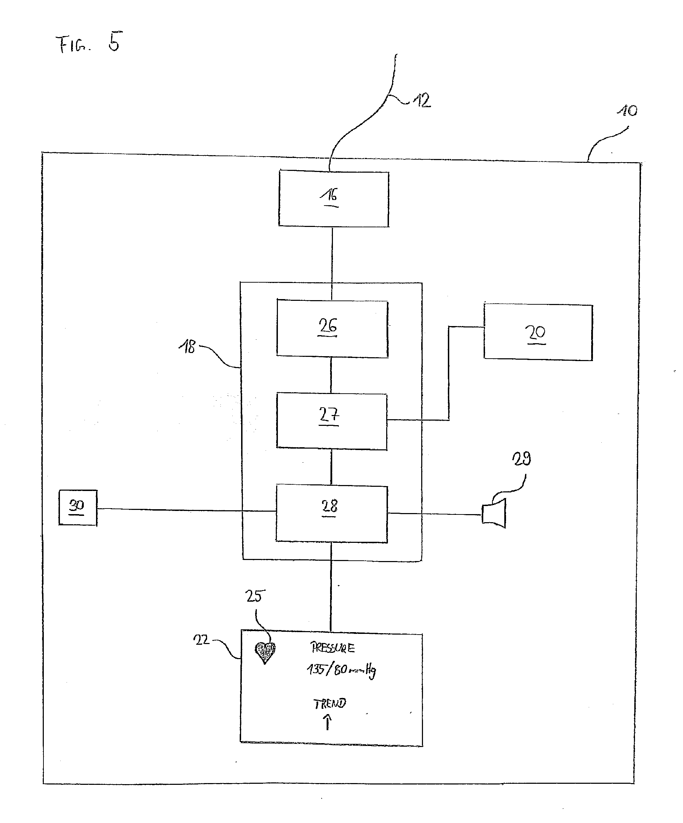

Figure 5 shows a schematic representation of an embodiment of the blood pressure measurement device 10 of the present invention. Pressure sensor 16 is connected to cuff 14 by means of flexible tube 12. Pressure oscillations are measured by pressure sensor 16 and delivered to the processor 18. In a first step 26, processor 18 determines diastolic and systolic pressure values from the measured oscillations. In a second step 27, the measured pressure values are compared with previously measured values stored in memory 20 and the blood pressure trend is calculated. In a third step 28, the pressure trend and actual pressure values are compared to predefined warning criteria, depending on the warning mode selected through switching means 30. If the values meet the criteria, a warning is indicated. The warning may be in the form of a special symbol 25 on display 22, such as a blinking heart, and/or an acoustic signal emitted by signalling means 29, such as a series of beeps.

In a first warning mode, a warning is indicated if the determined blood pressure trend shows increasing values over a specific period of time, e.g. for at least 20 days.

In a second warning mode, a warning is indicated if blood pressure values show a high increase over a short period of time. E.g. if the blood pressure shows an increase of more than 30 mmHg systolic and/or more than 15 mmHg diastolic in a period of less than two days, a warning is indicated. This can draw the attention of the patient to a possible medical condition, e.g. pre-eclepsia.

Switching means 30 may be a mechanical or an electrical switch. Alternatively the switching may also be done by means of any input button or switch already present on the device.

In a further embodiment, the blood pressure measurement device includes an erase function which deletes the most recent blood pressure measurement. Such a feature is useful if a different patient has unintentionally used the blood pressure measurement device of another patient. It is also envisaged that a warning is shown or that no trend is shown if the blood pressure values recorded by one device are too variable because this would indicate that a different patient had unintentionally used the device.

## Claims

**1.** A blood pressure measuring device (10) for measuring the blood pressure of a patient, comprising:
a storage means (20) for storing results of consecutive blood pressure measurements
a processor (18) for determining if there is a trend in the blood pressure measurements
a display (22) for displaying the trend in the blood pressure measurements.

**2.** A blood pressure measuring device (10) according to claim 1, wherein said blood pressure measuring device (10) is adapted to record one blood pressure measurement daily.

**3.** A blood pressure measuring device (10) according to claim 1, wherein said blood pressure measuring device (10) is adapted to record several blood pressure measurements during a day, and
the processor (18) is adapted to determine a daily average blood pressure measurement from said several blood pressure measurements and stores this result, and to determine the trend in the blood pressure measurements based on these daily average blood pressure measurements.

**4.** A blood pressure measuring device (10) according to one of claims 2 or 3, wherein said blood pressure measuring device (10) is adapted to record the daily blood pressure measurement(s) automatically.

**5.** A blood pressure measuring device (10) according to one of claims 2 or 3, wherein said blood pressure measuring device (10) is adapted to be user-activated to record the daily blood pressure measurement(s).

**6.** A blood pressure measuring device (10) according to one of the claims 1 to 5, wherein the processor is adapted to compare the results of a predetermined number of the daily blood pressure measurements in order to determine if there is a trend in those daily blood pressure measurements.

**7.** A blood pressure measuring device (10) according to claim 6, wherein the processor is adapted to compare the determined trend, when represented as a linear trend line over time, with a predetermined threshold gradient in order to determine if there is a trend in the blood pressure measurements.

**8.** A blood pressure measuring device (10) according to claim 7, wherein the minimum predetermined number of the daily blood pressure measurements is at least 10, preferably 14.

**9.** A blood pressure measuring device (10) according to one of the claims 1 to 5, wherein the processor is adapted to compare the result of the current blood pressure measurement with the result of at least one previous stored blood pressure measurement in order to determine if there is a trend in the blood pressure measurements.

**10.** A blood pressure measuring device (10) according to any of claims 1 to 6, wherein the processor (18) is adapted to compare at least the two most recent stored consecutive blood pressure measurements and the current blood pressure measurement in order to determine a trend in the blood pressure measurements.

**11.** A blood pressure measuring device (10) according to any of claims 1 to 6, wherein the processor (18) is adapted to compare all stored blood pressure measurements and the current consecutive blood pressure measurement in order to determine a trend in the blood pressure measurements.

**12.** A blood pressure measuring device (10) according to one of claims 10 or 11, further comprising a first switching means for switching between the different measurement modes.

**13.** A blood pressure measuring device (10) according to any of claims 6 to 12, wherein the processor (18) is adapted to indicate a warning if the trend is continuously increasing over a predetermined period of time, preferably at least 20 days, when the blood pressure measuring device (10) is operating in a first warning mode.

**14.** A blood pressure measuring device (10) according to any of claims 6 to 12, wherein the processor (18) is adapted to indicate a warning if the determined trend indicates a raise of systolic pressure of at least 30 mmHg and/or a raise of diastolic pressure of at least 15 mmHg for a period of less than 2 days, when the blood pressure measuring device (10) is operating in a second warning mode.

**15.** A blood pressure measuring device (10) according to claims 13 or 14, further comprising an second switching means (30) for switching between the first and the second warning mode.

**16.** A blood pressure measuring device (10) according to one of the claims 1 to 15, wherein the processor is adapted to display an increasing blood pressure trend by a first symbol (24a) and a decreasing blood pressure trend by a second symbol (24b) and a consistent blood pressure trend by a third symbol or no symbol (24c).

**17.** A blood pressure measuring device (10) according to claim 16, wherein the first symbol (24a) is an arrow pointing substantially to the top of the device and the second symbol (24b) is an arrow pointing substantially to the bottom of the device and the third symbol (24c) is an arrow pointing substantially across the device.

**18.** A blood pressure measuring device (10) according to any proceeding claim, further comprising a delete function which allows the most recent stored blood pressure measurement to be deleted.

**19.** A method for determining a cardiovascular risk factor of a patient, comprising the steps of:
recording a blood pressure measurement of the patient at a plurality of measurement times
storing consecutive blood pressure measurements
determining whether there is a trend in the blood pressure measurements,
if a trend is found to be present, displaying the trend in the blood pressure measurements.

**20.** A method according to claim 19, wherein the step of recording comprises recording one blood pressure measurement daily.

**21.** A method according to claim 19, wherein the step of recording comprises
recording several blood pressure measurements during a day, and
determining a daily average blood pressure measurement from said several blood pressure measurements and storing this result, and
determining the trend in the blood pressure measurement results based on these daily average blood pressure measurement results.

**22.** A method according to one of claims 20 or 21, wherein the step of recording comprises recording the daily blood pressure measurement(s) automatically.

**23.** A method according to one of claims 20 or 21, wherein the step of recording the daily blood pressure measurement(s) is user-activated.

**24.** A method according to one of claims 19 to 23, wherein the step of determining whether there is a trend in the blood pressure measurements, involves comparing the results of a predetermined number of the daily blood pressure measurements.

**25.** A method according to one of claims 19 to 24, wherein the step of comparing involves representing the results as a linear trend line over time and comparing this with a predetermined threshold gradient in order to determine if there is a trend in the blood pressure measurements.

**26.** A method according to one of claims 24 or 25, wherein the minimum predetermined number of the daily blood pressure measurements is 14.

**27.** A method according to one of claims 19 to 24, wherein the step of comparing comprises comparing the result of the current blood pressure measurement with the result of at least one previous stored blood pressure measurement in order to determine if there is a trend in the blood pressure measurements.

**28.** A method according to one of the claims 19 to 27, wherein the step of displaying the trend comprises indicating an increasing blood pressure trend by displaying a first symbol (24a) and indicating a decreasing blood pressure trend by displaying by a second symbol (24b) and indicating a consistent blood pressure trend by displaying a third symbol (24c).

**29.** A method according to any of claims 19 to 24, wherein the step of comparing comprises comparing at least the two most recent stored consecutive blood pressure measurements and the current blood pressure measurement in order to determine a trend in the blood pressure measurements.

**28.** A method according to any of claims 19 to 24, wherein the step of comparing comprises comparing all stored blood pressure measurements and the current consecutive blood pressure measurement in order to determine a trend in the blood pressure measurements.

**29.** A method according to any of claims 19 to 28, wherein the method further comprises comparing the determined trend and blood pressure values with a predefined criterion and to indicate a warning on display 22 if this criterion is met.

**30.** A method according to claim 29, wherein said predefined criterion is an increasing trend over a specified period of time, preferably 20 days.

**31.** A method according to claim 29, wherein said predefined criterion is an increase of the systolic pressure of at least 30 mmHg and/or an increase of diastolic pressure of at least 15 mmHg over a period of less than two days.
